# EUROPEAN PATENT APPLICATION

(11) **EP 2 127 605 A1**
(43) Date of publication of application: **02.12.2009**
(21) Application number: 09161450.3
(22) Date of filing: 28.05.2009
(51) Int. Cl.: A61B 17/12

(54) **Aneurysm treatment system**

(30) Priority: 30.05.2008 US 130292
(71) Applicant: Neurovasx, Inc., Maple Grove, MN 55369 (US)
(72) Inventor: Timko, Eric, Wayzata, MN 55391 (US); Calabria, Marie F., Plymouth, MN 55446 (US); Lee, Jeffrey A., Plymouth, MN 55447 (US)
(74) Representative: Curley, Donnacha John

(57) **Abstract**

An aneurysm treatment system includes a sleeve with a breach or a breachable facet disposed therein. The facet allows a guide structure to breach the sleeve and to deliver a portion of the sleeve continuously to an aneurysm.

## Description

### COPYRIGHT

A portion of the disclosure of this patent document contains material that is subject to copyright protection. The copyright owner has no objection to the facsimile reproduction by anyone of the patent document or the patent disclosure, as it appears in the Patent and Trademark Office patent files or records, but otherwise reserves all copyright rights whatsoever. The following notice applies to the products, processes, and data as described below and in the tables that form a part of this document: Copyright 2008, Neurovasx, Inc. All Rights Reserved.

### Background

An aneurysm is a balloon-like swelling in a wall of a blood vessel. An aneurysm results in weakness of the vessel wall in which it occurs. This weakness predisposes the vessel to tear or rupture with potentially catastrophic consequences for any individual having the aneurysm. Vascular aneurysms are a result of an abnormal dilation of a blood vessel, usually resulting from disease and/or genetic predisposition that can weaken the arterial wall and allow it to expand. Aneurysm sites tend to be areas of mechanical stress concentration so that fluid flow seems to be the most likely initiating cause for the formation of these aneurysms.

An aneurysm in a cerebral circulation tends to occur in an anterior communicating artery, posterior communicating artery, and a middle cerebral artery. The majority of these aneurysms arise from either curvature in the vessels or at bifurcations of these vessels. Cerebral aneurysms are most often diagnosed by the rupture and subarachnoid bleeding of the aneurysm.

Cerebral aneurysms are most commonly treated in open surgical procedures where the diseased vessel segment is clipped across the base of the aneurysm.
While considered to be an effective surgical technique, particularly considering an alternative that may be a ruptured or re-bleed of a cerebral aneurysm, conventional neurosurgery suffers from a number of disadvantages. The surgical procedure is complex and requires experienced surgeons and well-equipped surgical facilities. Current treatment options for cerebral aneurysm fall into two categories, surgical and interventional.

### BRIEF DESCRIPTION OF THE DRAWINGS

To depict the manner in which the embodiments are obtained, a more particular description of embodiments briefly described above will be rendered by reference to exemplary embodiments that are illustrated in the appended drawings. Understanding that these drawings depict only typical embodiments that are not necessarily drawn to scale and are not therefore to be considered to be limiting of its scope, the embodiments will be described and explained with additional specificity and detail through the use of the accompanying drawings in which:
FIG. 1 is a perspective elevation of a device for occluding an aneurysm according to an embodiment;
FIG. 2 is an elevational cross section of a aneurysm fill sleeve according to an embodiment;
FIG. 3 is an elevational cross section of a aneurysm fill sleeve according to an embodiment;
FIG. 4 is an elevational cross section of a aneurysm fill sleeve according to an embodiment;
FIG. 5 is an elevational cross section of a aneurysm fill sleeve according to an embodiment;
FIG. 6 is a schematic depiction of a system for occluding an aneurysm according to an embodiment;
FIG. 7a is a cross-sectional elevations and a partial schematic of a method of treating an aneurysm according to an embodiment;
FIG. 7b illustrates the aneurysm fill material depicted in FIG. 7a after further processing according to an embodiment; and
FIG. 8 is a method flow diagram for occluding an aneurysm according to an embodiment.

### DETAILED DESCRIPTION

Although detailed embodiments are disclosed herein, it is to be understood that the disclosed embodiments are merely exemplary of the invention that may be embodied in various and alternative forms. Specific structural and functional details disclosed herein are not to be interpreted as limiting, but merely as a basis for teaching one skilled in the art to variously employ the aneurysm treatment system embodiments. Throughout the drawings, like elements are given like numerals.

Referred to herein are trade names for materials including, but not limited to, polymers and optional components. The inventors herein do not intend to be limited by materials described and referenced by a certain trade name. Equivalent materials (e.g., those obtained from a different source under a different name or catalog (reference) number to those referenced by trade name) may be substituted and used in the methods described and claimed herein. All percentages and ratios are calculated by weight unless otherwise indicated. All percentages are calculated based on the total composition unless otherwise indicated. All component or composition levels are in reference to the active level of that component or composition, and are exclusive of impurities, for example, residual solvents or byproducts, which may be present in commercially available sources

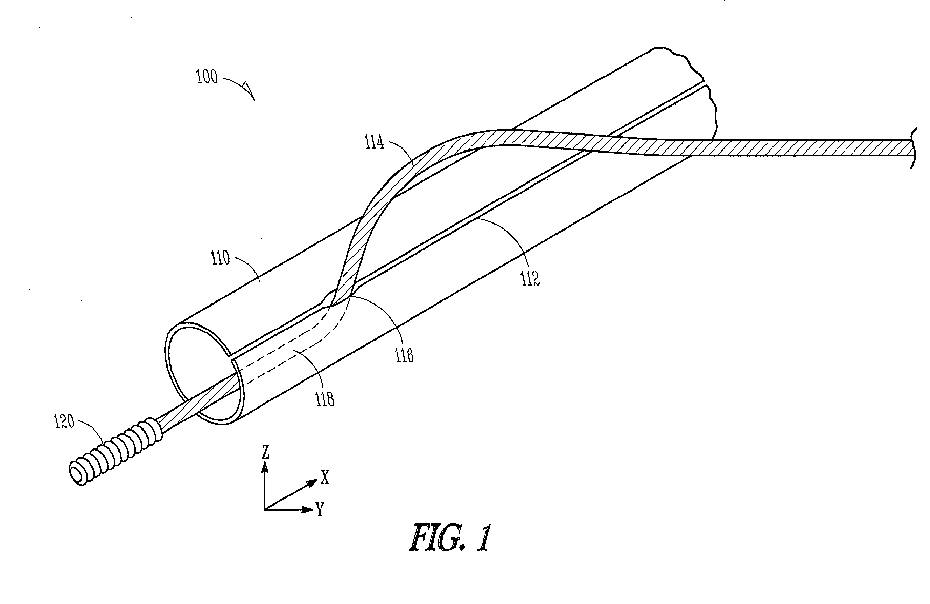

FIG. 1 is a perspective elevation of a device 100 for occluding an aneurysm according to an embodiment. A sleeve 110 includes a facet 112 such as a continuous, longitudinal slit therein according to an embodiment. In an embodiment, the facet 112 is substantially linearly configured along the length of the sleeve 110 as illustrated. In an embodiment, the facet 112 may be substantially non-linearly configured along the length of the sleeve 110 as illustrated so as to cause the sleeve to have more isotropic collapsing qualities to allow formation of an isotropic aneurysm fill material that conforms to the dimensions of a given aneurysm.

A guide structure 114 is shown as breaching the sleeve wall at the facet 112.
The guide structure 114 may be an insulated wire according to an embodiment. The guide structure 114 breaches the sleeve 110 at a location 116 in the facet 112. In an embodiment, the facet 112 is a continuous slit that allows the location 116 to be determined by the location of the guide structure 114 as it slips through the facet 112. The guide structure 114 is depicted in phantom lines 118 where it has breached the facet 112.

A detacher 120 is affixed to the guide structure 114 at a terminal end. In an embodiment, the detacher 120 is a resistor coil that heats under resistive heating by passing an electrical current through the guide structure 114 to complete an electrical circuit. In an embodiment, the guide structure 114 is a composite wire in an electrical circuit with the detacher 120 being located somewhere within the circuit. The detacher 120 may also be referred to as the head zone 120 of the guide structure 114.

The sleeve 110 is depicted as including a substantially circular cross section when viewed in the Y-Z plane. In an embodiment, the sleeve 110 may have a cross section other than circular.

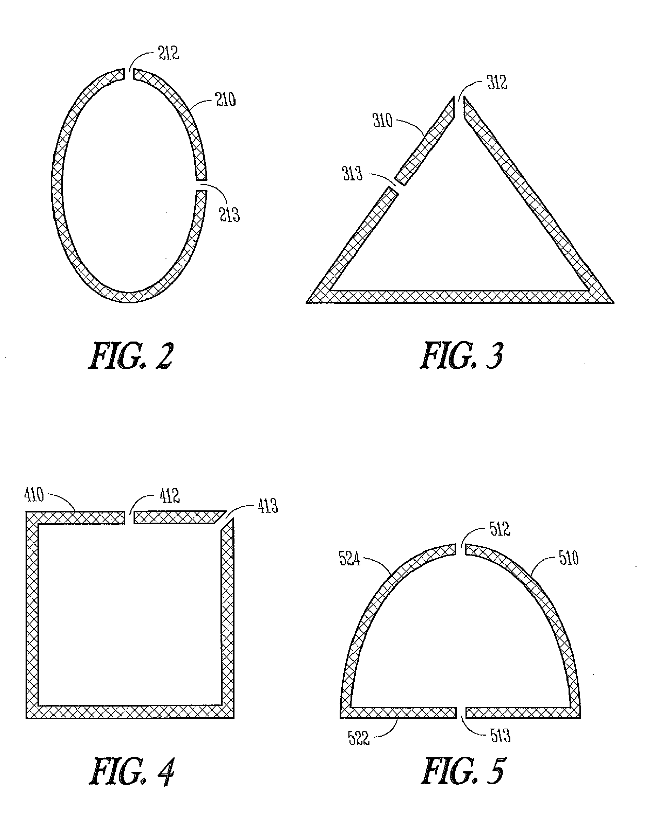

In an embodiment, the sleeve has an eccentric cross section such as is depicted in FIG. 2. The sleeve 210 shows two possible locations of the facet 212 and facet 213, although only one facet is present in a given embodiment. The facet 212 or the facet 213 may be located somewhere between the illustrated possible facet locations for an eccentric cross-section sleeve 210.

In an embodiment, the sleeve has a trigonal cross section such as is depicted in FIG. 3. The sleeve 310 shows two possible locations of the facet 312 and facet 313, although only one facet is present in a given embodiment. The facet 312 or the facet 313 may be located somewhere between the illustrated locations for a trigonal cross-section sleeve 310.

In an embodiment, the sleeve has a quadrilateral cross section such as is depicted in FIG. 4. The sleeve 410 shows two possible locations of the facet 412 and facet 413, although only one facet is present in a given embodiment. The facet 412 or the facet 413 may be located somewhere between the illustrated locations for a quadrilateral cross-section sleeve 410.

In an embodiment, the sleeve 510 has eccentric cross section that is a combination of a rectilinear surface 522 and a curvilinear surface 524 as depicted in FIG. 5. The sleeve 510 shows two possible locations of the facet 512 and facet 513, although only one facet is present in a given embodiment. The facet 512 or the facet 513 may be located somewhere between the illustrated locations for a combination rectilinear-curvilinear cross-section sleeve 510.

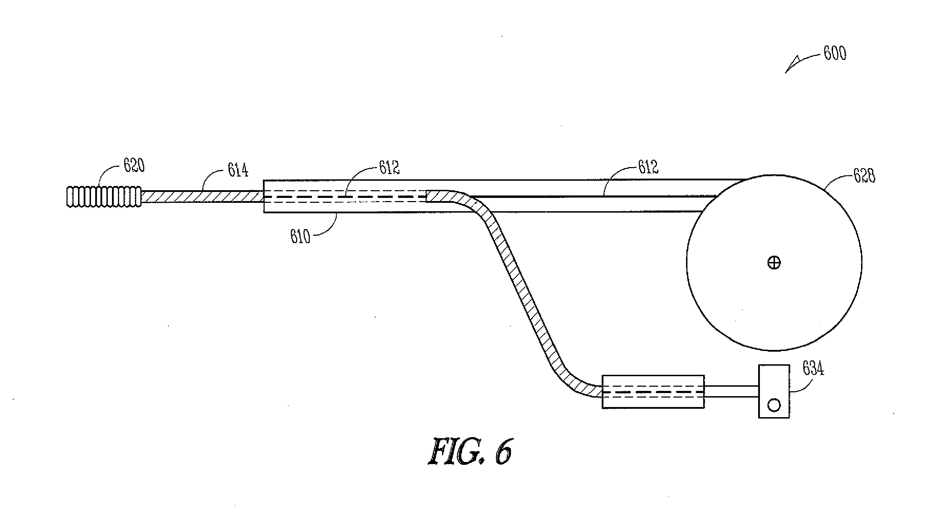

It is now clear that one of several cross sections may be selected to facilitate deployment of a portion of a given sleeve into an aneurysm. FIG. 6 is a schematic depiction of a system for occluding an aneurysm according to an embodiment. A sleeve 610 that includes a facet 612 such as a longitudinal slit 612, coupled with a guide structure 614, such as a wire with a detacher 620. The detacher may also be referred to as a head zone 620 of the guide structure 614. The sleeve 610 may be fed from a sleeve spool 628. The guide structure is connected via a connector cable to a power supply 634 for activating the heating process.

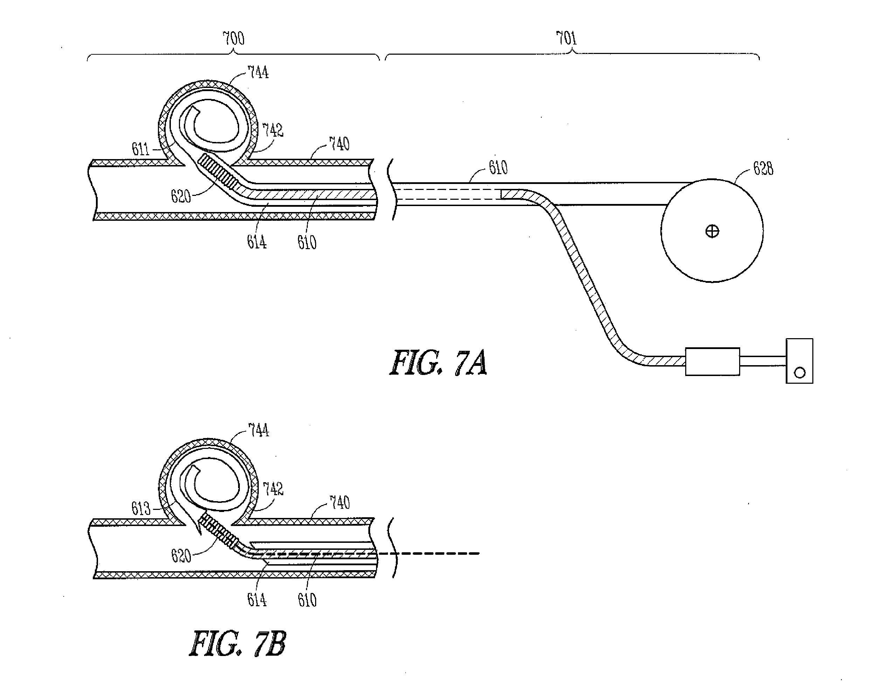

FIGs. 7a and 7b are cross-sectional elevations of a method of occluding an aneurysm according to an embodiment. At 700, a biolumen 740 is depicted that includes an aneurysm neck 742 and an aneurysm 744. At 701, a schematic of a system for occluding an aneurysm is depicted. The schematic depiction of a system for occluding an aneurysm 600 taken from FIG. 6 is used as an illustrative and nonlimiting example embodiment.

As illustrated in FIG. 7a, a sleeve 610 is depicted at 701 as a sheath over a guide structure 614. The sleeve 610 depicted at 701 is depicted at 700 inside the bio lumen 740 as an accrued sleeve mass 611 within the aneurysm 744. The head zone 620 of the guide structure is configured to assist the accrued sleeve mass 611 in terminating within the aneurysm 744.

FIG. 7b illustrates the aneurysm fill material depicted in FIG. 7a after further processing according to an embodiment. The accrued sleeve mass 611 (FIG. 7a) has been severed from the sleeve 610 by use of the head zone 620. The accrued sleeve mass 611 is depicted as an aneurysm fill 613. In this embodiment, the head zone 620 has been heated at or above a temperature at which the sleeve 610 withstands at least one of physical or chemical disintegration. Consequently, the aneurysm fill 613 has been severed from the balance of the sleeve 610.

The sleeve 610 is processed, forms the accrued sleeve mass 611, and by severing a portion thereof, forms the aneurysm fill 613 within the aneurysm 744.

The aneurysm treatment system provides for continuous delivery of aneurysm fill 613 into the aneurysm 744 over the guide structure 614, while allowing for detachment of the aneurysm fill 613 to occur anywhere along the length of the sleeve 610.

In an embodiment, the aneurysm fill 613 includes a mesh material that facilitates occlusion of an aneurysm. Such aneurysm-occlusion mesh material may have a pore size that facilitates *in vivo* fibrotic cell growth in the aneurysm fill 613. In an example embodiment, the aneurysm fill 613 includes a polymeric material such as polyethylene.

In an embodiment, the aneurysm fill 613 includes a hydrogel foam portion that can be entrained in a polyethylene matrix. In an embodiment, the hydrogel is swellable and has a swell ratio of 10:1-2:1. The foam provides a desirable surface for rapid cell ingrowth. The hydrogel foam or other filler material is shapable at the aneurysm neck to form a smooth, closed surface at a neck of an aneurysm.

Swellable materials for use as the aneurysm fill 613 include acrylic-based materials according to an embodiment. For example, the aneurysm fill 613 is deployed within an aneurysm where least some active portions of the aneurysm fill 613 have a time-dependent rate of dissolution such that swellable materials are encapsulated, but after deployment in a target animal, the swellable materials may be contacted with *in vivo* fluids that dissolve encapsulation layer(s).

Where hydrogel is part of the aneurysm fill 613, the hydrogel deployed within the aneurysm fill 613 may be stiffened as a consequence of an increased degree of crosslinkage as compared to other portions of the aneurysm fill 613. While a hydrogel is described, it is understood that other biocompatible, swellable materials are suitable for use in selected embodiments.

Other materials include acetates, such as cellulose acetate or polyvinylacetate, for the aneurysm fill 613. Other materials include alcohols, such as ethylene vinyl alcohol copolymers, for the aneurysm fill 613. Other materials include nitriles, such as polyacrylonitriles, for the aneurysm fill 613. Other materials include cellulose acetate butyrate, nitrocellulose, copolymers of urethane/carbonate, copolymers of styrene/maleic acid, or mixtures thereof for the aneurysm fill 613. In particular, a hydrogel/polyurethane foam is usable in for the aneurysm fill 613.

In an embodiment, the aneurysm fill 613 is seeded with time-release sclerotheraputic substances that may cause an aneurysm to close or to become growth-static. In an embodiment, the aneurysm fill 613 includes an integrin substance.

In an embodiment, the aneurysm fill 613 includes a polymer-based, coil-biased structure that favors forming an isotropic mass after deployment within an aneurysm. In an embodiment, the bias is not coil-based, but merely non-linear biased to facilitate forming an isotropic mass. In an embodiment, the aneurysm fill 613 is fabricated with soft biocompatible polymers such as PTFE, urethanes, polyolefins, nylons, and the like. In an embodiment, the aneurysm fill 613 includes hollow coils. The aneurysm fill 613 may also be fabricated from biodegradable materials such as PLA, PGA, PLGA, polyanhydrides, and other similar biodegradable materials. Use of biodegradable materials provokes a wound healing response and concomitantly eliminates a mass effect of the aneurysm shield anchor over time.

The material of the aneurysm fill 613 described herein may be one or more of polymeric and polymeric hybrids such as PEBAX, Grilamids, polyester, and silica. Aneurysm-occlusion structure materials may also include reabsorbables such as PGLA, PEG, PGLA, and base polymer. Other aneurysm-occlusion structure materials may include textiles such as rayon, nylon, silk, Kyeon, Kevlar, and cotton. Other aneurysm-occlusion structure materials may include biopolymers such as collagen, filaments, and coated polymeric material. Other aneurysm-occlusion structure materials may include elastomers such as urethanes, silicones, nitriles, Teco Flux, carbothane, and silicone hybrids.

In an embodiment, the aneurysm fill 613 may include a coating material thereon. The coating material may be disposed continuously or discontinuously on either the inner or outer surface of the aneurysm fill 613. The coating material can be one or more of a biologically inert material (e.g., to reduce the thrombogenicity of the prosthesis), a medicinal composition which leaches into the wall of the body passageway after implantation (e.g., to provide anticoagulant action, to deliver a pharmaceutical to the body passageway) and the like.

For some embodiments, the aneurysm fill 613 is provided with a biocompatible coating to minimize adverse interaction with the walls of the body vessel and/or with the liquid, usually blood, flowing through the vessel. For some embodiments, the coating is a polymeric material, which is provided by applying to the prosthesis a solution or dispersion of preformed polymer in a solvent and removing the solvent. Non-polymeric coating material may alternatively be used. Suitable coating materials, for instance polymers, may be polytetraflouroethylene or silicone rubbers, or polyurethanes that are known to be biocompatible. In an embodiment the polymer has zwitterionic pendant groups, generally ammonium phosphate ester groups, such as phosphoryl choline groups or analogues thereof

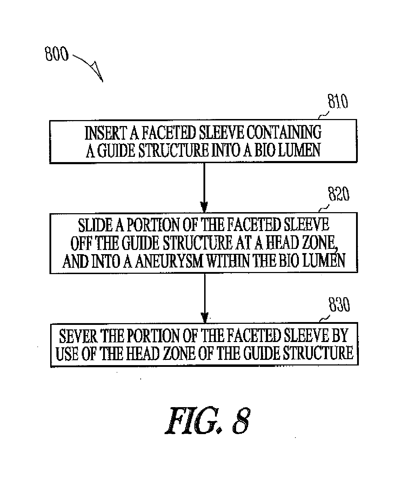

FIG. 8 is a method flow diagram 800 for occluding an aneurysm according to an embodiment.

At 810, the method includes inserting a faceted sleeve over a guide structure into a bio lumen.

At 820, the method includes sliding a portion of the faceted sleeve off the guide structure at a head zone thereof and into an aneurysm with the bio lumen.

At 830, the method includes severing a portion of the faceted sleeve by use of the head zone to form an aneurysm fill.

The Abstract is provided to comply with 37 C.F.R. §1.72(b) requiring an abstract that will allow the reader to quickly ascertain the nature and gist of the technical disclosure. It is submitted with the understanding that it will not be used to interpret or limit the scope or meaning of the claims.

In the foregoing Detailed Description, various features are grouped together in a single embodiment for the purpose of streamlining the disclosure. This method of disclosure is not to be interpreted as reflecting an intention that the claimed embodiments of the invention require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate preferred embodiment.

It will be readily understood to those skilled in the art that various other changes in the details, material, and arrangements of the parts and method stages which have been described and illustrated in order to explain the nature of this invention may be made without departing from the principles and scope of the invention as expressed in the subjoined claims.

## Claims

1. A device for occluding an aneurysm comprising:
a sleeve, wherein the sleeve includes a facet therein to allow an object to breach the sleeve, and wherein the sleeve includes materials to respond within an animal to fill an aneurysm; and
a guide structure including an elongate body and a detacher, wherein the sleeve and the guide structure are slidingly coupled through the facet.

2. The device of claim 1, wherein the sleeve has a cross-sectional form factor selected from circlar, eccentric circlar, rectilinear, and combination curvilinear and rectilinear.

3. The device of claim 1, wherein the facet is a longitudinal slit disposed therein.

4. The device of claim 1, wherein the guide structure is an electrically conductive body and wherein the detacher is an electirically resistive heater element.

5. The device of claim 1, wherein the sleeve includes a polymer material, wherein the facet is a longitudinal slit disposed therein, wherein the guide structure is an electrically conductive body, and wherein the detacher is an electrically resistive heater element.

6. The device of claim 1, wherein the sleeve includes a non-linear bias.

7. A system for occluding an aneurysm comprising:
a sleeve, wherein the sleeve includes a facet therein to allow a solid object to breach the sleeve, and wherein the sleeve includes materials to respond within an animal to fill an aneurysm;
a guide structure including an elongate body and a detacher, wherein the sleeve and the guide structure are slidingly coupled through the facet; and
an electrical apparatus to couple with the guide structure and to heat the detacher.

8. The system of claim 7 further including an electrical coupling to connect the guide structure to the electrical apparatus.

9. The system of claim 7, wherein the sleeve facet includes a longitudinal slit.

10. A method of treating an aneurysm comprising:
deploying a sleeve slidingly along a guide structure and into a bio lumen, wherein the sleeve includes a facet therein to allow the guide structure to breach the sleeve;
directing the sleeve into an aneurysm within the bio lumen and at a detacher of the guide structure; and
severing a portion of the sleeve at a head zone of the guide structure.

11. The method of claim 10, wherein directing the sleeve into the aneurysm is carried out under conditions to cause the sleeve to form an isotropic mass within the aneurysm.

12. The method of claim 10, wherein directing the sleeve into the aneurysm is carried out under conditions to cause the sleeve to form an isotropic mass within the aneurysm, wherein the head zone of the guide structure locally causes at least one of a physical change and a chemical change to the sleeve.

13. The method of claim 10, wherein the guide structure includes a fixed, obtuse angle at detatcher, and wherein directing the sleeve into the aneurysm is facilitated by the fixed, obtuse angle.

14. The method of claim 10, wherein severing a portion of the sleeve includes electrically heating the head zone under conditions to cause the sleeve to locally fragment under heat load.

15. The method of claim 10, wherein the guide structure includes a fixed, obtuse angle at the head zone thereof, and wherein directing the sleeve into the aneurysm is facilitated by the fixed, obtuse angle, wherein directing the sleeve into the aneurysm is carried out under conditions to cause the sleeve to form an isotropic mass within the aneurysm, wherein the head zone of the guide structure locally causes at least one of a physical change and a chemical change to the sleeve.
